# EUROPEAN PATENT APPLICATION

(11) **EP 1 829 491 A1**
(43) Date of publication of application: **05.09.2007**
(21) Application number: 06251152.2
(22) Date of filing: 02.03.2006
(51) Int. Cl.: A61B 17/42, A61B 17/34

(54) **Transvaginal tube**

(71) Applicant: Hopefar International Limited, Road Town, Tortola (VG)
(72) Inventor: Spiritos, Nicholas Michael, Las Vegas 89109 Nevada (US); Yang, Chih-Hao, Chang Hua City (TW); Yang, Ku-Yueh Wu, Chang Hua City (TW)
(74) Representative: Mounteney, Simon James

(57) **Abstract**

A transvaginal tube (100) for laparoscopic surgery includes a tubular wall (10) defining a longitudinal axis (L) and including an outer end section (11), an insert end section (12), and an intermediate section (13) interconnecting the insert end section (12) and the outer end section (110. The insert end section (12) has a terminating end (123), and f lares to the terminating end (123) from a juncture (121) of the intermediate section (13) and the insert end section (12) so that the terminating end (123) has a cross-section larger than that of the juncture (121).

## Description

The invention relates to a transvaginal tube, more particularly to a transvaginal tube adapted for invasive laparoscopic surgery.

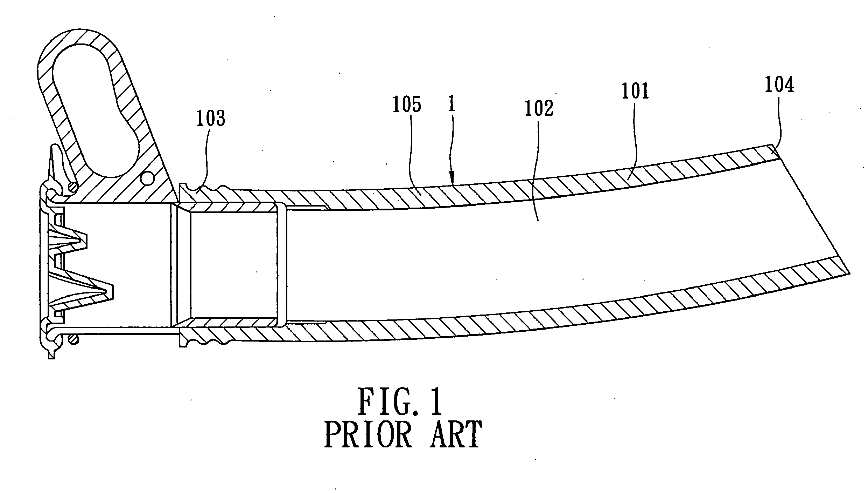

Figure 1 shows a transvaginal tube 1 for gynaecological laparoscopic surgical procedures described in USP 6, 572, 631 B1. The transvaginal tube 1 is mainly used to stretch the vagina of a patient for passage of a medical instrument through the transvaginal tube 1 into the body of the patient to perform a laparoscopic surgery. The transvaginal tube 1 is generally hollow, and has a tubular wall 101 that surrounds a tubular axis and that defines a channel 102. The tubular wall 101 has a proximal end 104 for extension into the vagina up to the cervix, a distal end 103 opposite to the proximal end 104 and disposed externally of the body of the patient, and a middle section 105 interposed between the proximal and distal ends 104, 103.

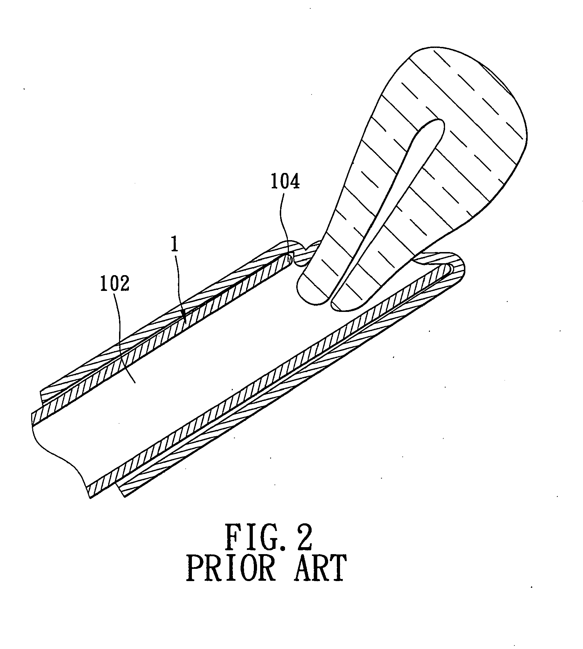

Referring to Figure 2, as the proximal end 104 of the transvaginal tube 1 is not designed to have a relatively large opening, in use, when a medical instrument inserted through the channel 102 to reach the uterus to perform a surgical operation, the operation is relatively difficult to conduct due to limited available room.

Furthermore, if the transvaginal tube 1 is formed from relatively soft silicone rubber or TPR (thermoplastic rubber) for the sake of the patient's comfort, the transvaginal tube 1 is likely to deform after being inserted into the vagina, which may even obstruct smooth performance of the surgical procedure. If the tubular wall 101 is formed to have a relatively large thickness in order to prevent undesirable deformation of the transvaginal tube 1, this may, however, results in discomfort on the patient's part.

In addition, since the cross-section of the tubular wall 101 is generally uniform and invariable, the transvaginal tube 1 is unable to fit different patients and needs to be made into different sizes.

Therefore, the main object of the present invention is to provide a transvaginal tube for laparoscopic surgery, which has a flared insert end section to provide more room for facilitating surgical operation.

According to this invention, a transvaginal tube for laparoscopic surgery includes a tubular wall defining a longitudinal axis and including an outer end section, an insert end section, and an intermediate section interconnecting the insert end section and the outer end section. The insert end section has a terminating end, and flares to the terminating end from a juncture of the intermediate section and the insert end section so that the terminating end has a cross-section larger than that of the juncture.

Other features and advantages of the present invention will become apparent in the following detailed description of the preferred embodiment with reference to the accompanying drawings, of which:
Figure 1 is a schematic sectional view of a conventional transvaginal tube;
Figure 2 is a fragmentary schematic view of the conventional transvaginal tube in situ;
Figure 3 is a sectional view of a preferred embodiment of a transvaginal tube according to this invention;
Figure 4 is a top view of the preferred embodiment;
Figure 5 is a schematic view of the preferred embodiment, showing how an insert end section is pressed and deformed; and
Figure 6 is a fragmentary schematic view illustrating the preferred embodiment in situ.

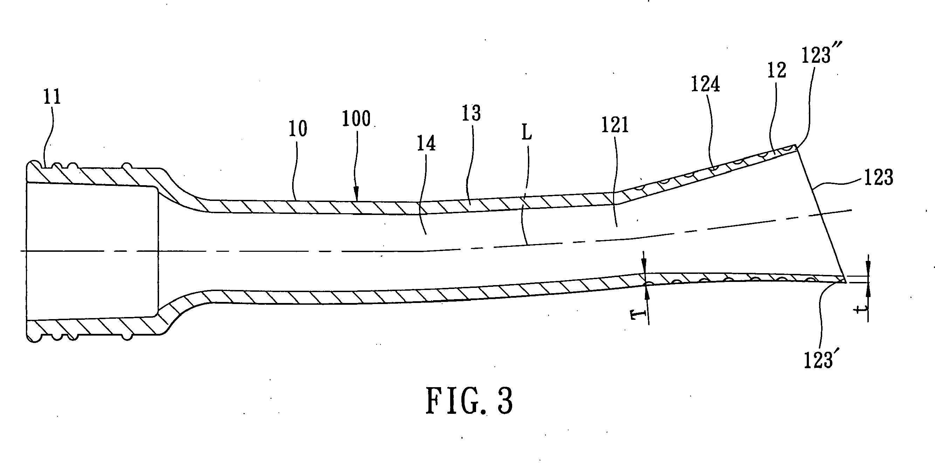

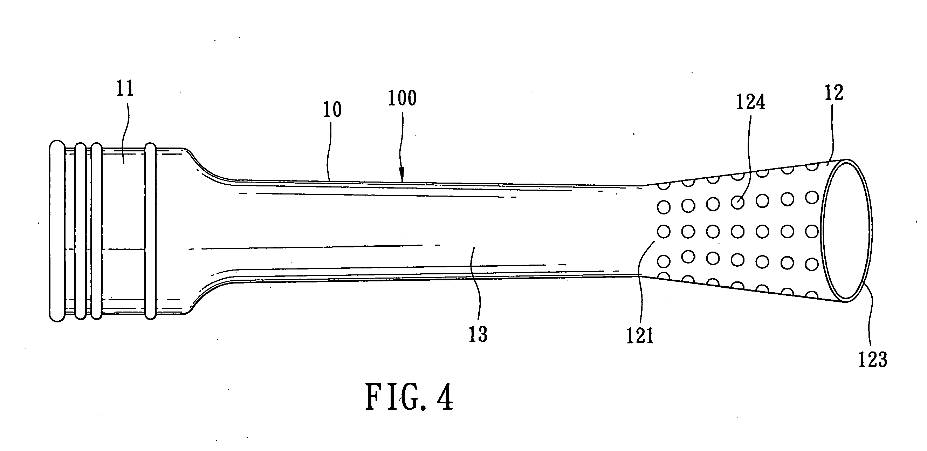

Referring to Figures 3 to 6, the preferred embodiment of a transvaginal tube 100 for laparoscopic surgery according to the present invention is adapted to be inserted into the vagina 200 of a patient to be proximate to the cervical neck 210 and the uterus opening 230 so as to enable inspection and surgical operation of the uterus 220. The transvaginal tube 100 is preferably formed from a material with resilience, such as silicone rubber and TPR (thermoplastic rubber). As shown, the transvaginal tube 100 includes a tubularwall 10 defining alongitudinalaxis (L) and confining a channel 14 therein. In this embodiment, the longitudinal axis (L) is curved. The tubular wall 10 includes an outer end section 11, an insert end section 12 opposite to the outer end section 11, and an intermediate section 13 interconnecting the insert end section 12 and the outer end section 11. The insert end section 12 has a terminating end 123 which lies in a plane that is non-normal to the longitudinal axis (L) and which has a top edge 123" and a bottom edge 123'. The insert end section 12 flares to the terminating end 123 from a juncture 121 of the intermediate section 13 and the insert end section 12 so that the terminating end 123 has a cross-section larger than that of the juncture 121. The tubular wall 10 has a wall thickness which is tapered toward the terminating end 123 from the juncture 121. That is, the wall thickness (T) of the tubular wall 10 at the juncture 121 is greater than the wall thickness (t) at the terminating end 123. Furthermore, the insert end section 12 includes a plurality of anti-slip depressions 124 formed in an outer surface thereof.

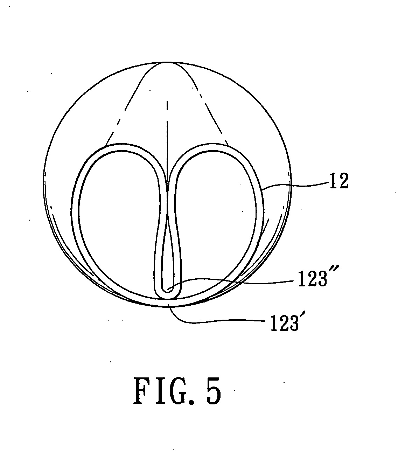

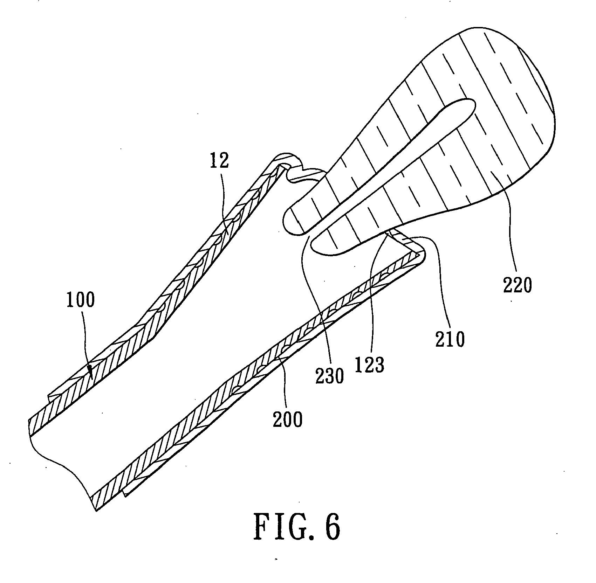

In use, referring to Figure 5, the insert end section 12 is pressed flat (i.e., by pressing the top edge 123" against the bottom edge 123'), with the lateral sides squeezed in, so that the insert end section 12 becomes smaller in cross-section to facilitate insertion thereof into the patient's vagina 200 for laparoscopic surgery. Referring to Figure 6, when the insert end section 12 reaches the cervical neck 210 of the patient, the insert end section 12 will expand due to the resilience thereof. As the terminating end 123 lies in a plane non-normal to the longitudinal axis (L), i.e., the terminating end 123 has an inclined or beveled profile, it can fit snugly around the cervical neck 210. Thus, medical personnel can perform laparoscopic surgery or the like using the transvaginal tube 100 of this invention.

In view of the construction of the transvaginal tube 100, this invention has the following advantageous effects:
1. The configuration of the flared insert end section 12 provides more room to facilitate surgical operation.
2. The beveled configuration of the terminating end 123 provides certain directionality when the insert end section 12 is squeezed for insertion into the vagina 200. Besides, the beveled terminating end 123 can fit snugly and relatively securely around the cervical neck 210.
3. As the wall thickness (T) of the tubular wall 10 at the juncture 121 is greater than the wall thickness (t) at the terminating end 123, the insert end section 12 can be pressed and deformed with relative ease, and can render the transvaginal tube 100 suitable for use in different patients.
4. The arrangement of the anti-slip depressions 124 helps prevent outward slippage of the transvaginal tube 100 during insertion thereof into the patient's vagina 200 while causing no discomfort to the patient.

## Claims

1. A transvaginal tube (100) adapted for laparoscopic surgery, **characterized by**:
a tubular wall (10) defining a longitudinal axis (L) and including an outer end section (11), an insert end section (12), and an intermediate section (13) interconnecting said insert end section (12) and said outer end section (11), said insert end section (12) having a terminating end (123) and flaring to said terminating end (123) from a juncture (121) of said intermediate section (13) and said insert end section (12) so that said terminating end (123) has a cross-sectionlargerthanthatof saidjuncture (121).

2. The transvaginal tube (100) as claimed in Claim 1, **characterized in that** said insert end section (12) lies in a plane that is non-normal to said longitudinal axis (L).

3. The transvaginal tube (100) as claimed in Claim 2, further **characterized in that** said tubular wall (10) has a wall thickness which is tapered toward said terminating end (123) from said juncture (121).

4. The transvaginal tube (100) as claimed in Claim 3, further **characterized in that** said longitudinal axis (L) is curved.

5. The transvaginal tube (100) as claimed in Claim 1, **characterized in that** said insert end section (12) includes a plurality of anti-slip depressions (124).
